# EUROPEAN PATENT APPLICATION

(11) **EP 4 537 776 A1**
(43) Date of publication of application: **16.04.2025**
(21) Application number: 24206019.2
(22) Date of filing: 11.10.2024
(51) Int. Cl.: A61B 18/14, A61B 5/00, H01B 7/28, H01B 7/29, A61B 18/00

(54) **PROTECTIVE SLEEVE FOR CATHETER ELECTRICAL CABLE**

(30) Priority: 13.10.2023 US 202318379823
(71) Applicant: Biosense Webster (Israel) Ltd., 2066717 Yokneam (IL)
(72) Inventor: VAN NIEKERK, Pieter E., Irvine, 92618 (US); TOBEY, Dustin R., Irvine, 92618 (US); EBRAHIMI, Babak, Irvine, 92618 (US)
(74) Representative: Carpmaels & Ransford LLP

(57) **Abstract**

A protective sleeve for shielding an electrical electrophysiology cable from blood and fluid splatter, comprising a distal member with a first through-hole, a proximal member with a second through-hole, and an elongated flexible tubular member therebetween through which the electrical cable can extend. When used with a catheter control handle, the sleeve can assume a stored configuration and a deployed configuration. The sleeve can be stored in a proximal end of the control handle and deployed from the proximal end of the catheter control handle. The sleeve can also be stored separately from the control handle and mounted onto the catheter control handle for deployment in extending over the electrical cable when electrical cable is connected to the catheter control handle. The sleeve can be readily disposed of along with the catheter control handle that is intended for single use.

## Description

### FIELD OF INVENTION

This invention relates to electrophysiology catheters, in particular, electrophysiology catheters used with electrical cables for relaying signals and power.

### BACKGROUND

Cardiac arrhythmia is irregular beating of the heart caused by aberrant electrical signals. Arrhythmias can reduce quality of life and carry increased risk of stroke and heart failure. Arrythmias can be located and identified via diagnostic catheters. These catheters can be used to create electroanatomical maps to help electrophysiologists understand the pathology and plan and deliver therapy which can include ablation via therapeutic catheters.

A conventional electrophysiology (EP) catheter, whether diagnostic or therapeutic, is connected to an advanced imaging system that utilizes electromagnetic technology to create real-time three-dimensional (3D) maps of a patient's cardiac structure. The system is designed to help electrophysiologists navigate the heart by generating an accurate 3D map, as well as pinpointing the exact location and orientation of catheters in the heart during diagnostic and therapeutic procedures. The system includes hardware and software modules that provide a variety of features and functions, including delivery of power and ablation energy to the catheter and processing of signals sensed and relayed by the catheter via an electrical cable that connects the catheter to the system. When the catheter is introduced into the patient's vasculature, blood and other bodily fluids can splash from the point of entry onto nearby devices and equipment. Whereas catheters are designed and manufactured for single use followed by disposal, electrical cables are intended for reuse between patients or procedures and therefore require sterilization which is a process that exposes the cables to harsh treatment, including, intense heat or chemicals, that can degrade the cables.

Accordingly, applicants recognized that there is a need to provide electrical cables with splashguard barrier and protection with the use of a disposable protective sleeve that can be readily mounted onto or conveniently stored in a catheter control handle and readily deployed to extend over the electrical cable connected thereto.

### SUMMARY OF THE DISCLOSURE

In some embodiments, a sleeve that provides splashguard protection for a reusable electrical cable connected to a medical probe or catheter intended for single use, comprises a distal member with a first through-hole, a proximal member with a second through-hole, and an elongated flexible tubular member that includes a distal end affixed to the distal member and a proximal end affixed to the proximal member. The tubular member includes a channel through which the cable extends. The channel is in communication with the first through-hole at the distal end and in communication with the second through-hole at the proximal end.

In some embodiments, the distal member of the sleeve is configured for attachment to a catheter control handle.

In some embodiments, the distal member and the proximal member of the sleeve are releasably coupled to each other.

In some embodiments, the sleeve further includes a releasable adhesive between the distal member and the proximal member.

In some embodiments, the distal member includes a threaded distal portion that is received in a proximal end of the catheter control handle.

In some embodiments, the tubular member is constructed of a flexible thin plastic film.

In some embodiments, the tubular member is fluidproof.

In some embodiments, each of the distal member and the proximal member has a generally circular cross-section configuration.

In some embodiments, a protective sleeve configured for use with a catheter control handle, comprises an elongated tubular member including a distal end, a proximal end and a flexible body portion between the distal end and the proximal end. The distal end is attached to the control handle, and the proximal end and the body portion extend proximally from the catheter control handle.

In some embodiments, the sleeve further includes an elastic loop member that extends circumferentially around the distal end of the sleeve and the control handle.

In some embodiments, the distal end of the tubular member includes a portion that wedges into a slit provided in the control handle.

In some embodiments, the tubular member is constructed of a flexible thin plastic film.

In some embodiments, a catheter control handle for use with an electrical cable, comprises a distal end electrical connector and a housing with a longitudinal axis and a proximal end that extends circumferentially around the electrical connector relative to the longitudinal axis. Also included is an elongated sleeve with a distal end, a proximal end, and a flexible tubular portion therebetween, where the distal end of the sleeve is affixed to the proximal end of the catheter control handle, and the sleeve is configured to assume a deployed configuration when the electrical cable is attached to the catheter control handle and a stored configuration when the electrical cable is detached from the catheter control handle.

In some embodiments, the stored configuration includes the tubular portion of the sleeve being collapsed within the proximal end of the housing.

In some embodiments, the deployed configuration includes the proximal end and the tubular portion of the sleeve extending proximally from the control handle.

In some embodiments, the electrical connector is configured to connect to an electrical cable, and the sleeve in the deployed configuration includes the tubular portion and the proximal end of the sleeve extending over at least a distal portion of the electrical cable.

In some embodiments, the proximal end includes an inner surface to which the distal end of the elongated sleeve is attached.

In some embodiments, the sleeve includes a construction material of a flexible thin plastic film.

In some embodiments, a sleeve for use with an electrical cable configured to be attached to a connector cable extending from a catheter, comprises an elongated tubular member that includes a distal end, a proximal end and a middle portion extending therebetween. The distal end of the tubular member is attached to a proximal end of the connector cable that is attached at its distal end to a control handle of the catheter such that the proximal end and the middle portion of the elongated tubular member extends proximally over the electrical cable when the distal end of the electrical cable is connected to a proximal end of the connector cable.

In some embodiments, the sleeve includes a construction material of a flexible thin plastic film.

### BRIEF DESCRIPTION OF THE DRAWINGS

These and other features and advantages of the embodiments of the invention will be better understood by reference to the following detailed description when considered in conjunction with the accompanying drawings. It is understood that selected structures and features have not been shown in certain drawings so as to provide better viewing of the remaining structures and features.
FIG. 1 is a top plan view of a protective sleeve covering an electrical cable that is connected to a catheter control handle, according to an embodiment.
FIG. 2 is a perspective view of a protective sleeve in a deployed configuration, extending proximally from a proximal end of a catheter control handle, according to an embodiment.
FIG. 3 is a perspective view of the protective sleeve of FIG. 2, in a stored configuration, collapsed inside the proximal end of the catheter control handle.
FIG. 4A is a top plan view of a protective sleeve in a stored configuration, with a proximal ring member positioned on a proximal end of a catheter control handle, according to an embodiment.
FIG. 4B is a top plan view of the protective sleeve of FIG. 4A, in a deployed configuration, with the proximal ring member and the protective sleeve extending over an electrical cable connected to the catheter control handle.
FIG. 4C is a top plan view of a protective sleeve and a proximal ring member with a threaded distal portion, in a deployed configuration, according to an embodiment.
FIG. 5 is a perspective view of a protective sleeve with its distal end mounted over an outer surface of a catheter control handle, according to an embodiment.
FIG. 6 is a perspective view of a protective sleeve with its distal end mounted over an outer surface of a catheter control handle, according to another embodiment.
FIG. 7A is a perspective view of a protective sleeve with its distal end mounted over an outer surface of a catheter control handle, according to yet another embodiment.
FIG. 7B is a detailed view of the catheter control handle of FIG. 7A.
FIG. 8A is a side elevation view of a sleeve with a distal member and a proximal member releasably adhered to each other.
FIG. 8B is a side elevation view of the sleeve of FIG. 8A, with the distal member and the proximal member detached from each other.
FIG. 8C is a cross-sectional view of the sleeve of FIG. 8B.
FIG. 8D is a top plan view of a catheter control handle configured for use with the sleeve of FIG. 8A, FIG. 8B and FIG. 8C in providing a splashguard for an electrical cable connected to the catheter control handle.
FIG. 9 is a top plan view of a catheter with an electrical connector cable to which a sleeve and an electrical cable protected by the sleeve are attached.

### DETAILED DESCRIPTION OF THE PREFERRED EMBODIMENTS

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71 % to 99%. In addition, as used herein, the terms "patient," "host," "user," and "subject" refer to any human or animal subject and are not intended to limit the systems or methods to human use, although use of the subject invention in a human patient represents a preferred embodiment.

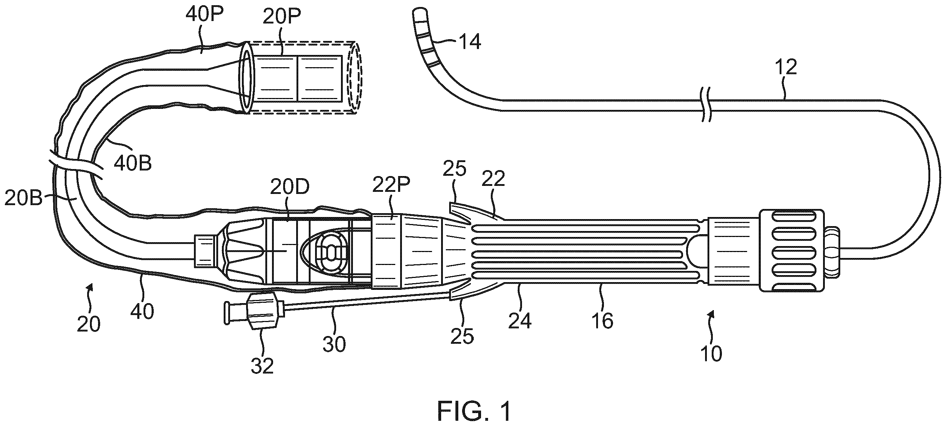

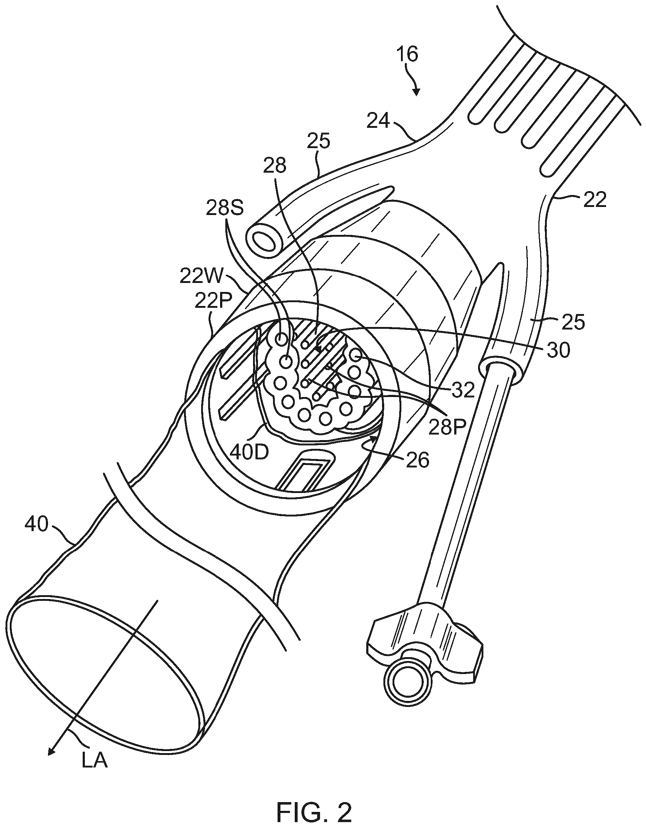

Referring to **FIG. 1** and **FIG. 2****,** an electrophysiology catheter 10 includes an elongated shaft 12, a distal end effector 14, and a control handle 16. The control handle 16 has a longitudinal axis LA defined by its length and is configured for connection with an electrical cable 20 that includes a distal connector 20D, a proximal connector 20P and an elongated cable body 20B extending therebetween. In some embodiments, the control handle 16 has a housing 22 which has a proximal end 22P with a circumferential wall 22W relative to the longitudinal axis LA. The wall 22W has an inner surface 26 that defines a cavity 30 and a proximal opening 32 that provides access to an electrical connector 28 situated in the cavity 30. The electrical connector 28 with pins 28P and sockets 28S is configured for connection with the distal connector 20D of the electrical cable 20 with corresponding sockets 20S and pins 20P. Outer surface 24 of the housing 22 may be formed with one or more lateral extensions or arms 25 to facilitate other connections to the control handle 16, for example, an irrigation tubing 30 connected to and luer hub 32.

Advantageously, an elongated protective sleeve 40 spans over the cable 20 as a splashguard against blood and other bodily fluids that may leak from a catheter entry point into the patient's body and otherwise contaminate the cable 20 and require the cable to undergo sterilization prior to use with another patient or in another procedure. The sleeve 40 has a distal end 40D, a proximal end 40P and an elongated tubular body 40B extending therebetween. The tubular body 40B has a pathway therethrough, with a distal opening at the distal end 40D and a proximal opening at the proximal end 40P. In the illustrated embodiment of **FIG. 1** and **FIG. 2****,** the distal end 40D of the sleeve 40 is affixed and attached circumferentially to the inner surface 26 of the distal wall 22W of the catheter housing, with the electrical connector 28 of the control handle 16 extending through the distal opening of the sleeve. When the connector 28 of the control handle 16 is connected to the distal connector 20D of the cable 20, the cable body 20B extends through the tubular body 40B with the tubular body circumferentially surrounding the cable body. The length of the tubular body 40B may be at least comparable to the length of the cable body 20B (as shown in solid lines in **FIG. 1****)**, if not also greater, being of a length sufficient to cover the proximal connector 20P (as shown in broken lines in **FIG. 1****).**

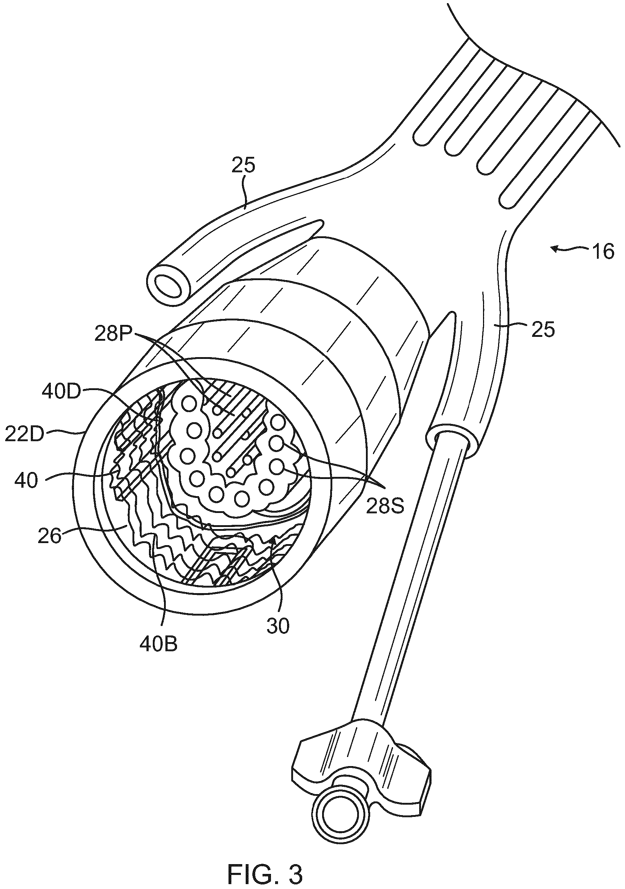

The sleeve 40 is configured to assume at least two configurations, namely, an expanded or deployed configuration as shown in **FIG. 1** and **FIG. 2****,** and a collapsed or stored configuration as shown in **FIG. 3****.** In the stored configuration of **FIG. 3****,** the sleeve 40 body is collapsed (e.g., tucked, folded or scrunched) into a generally toroidal or "donut" shape, with a through-hole," that fits in the cavity 30 of the housing 22, with the electrical connector 28 of the control handle 16 extending through the through-hole so that the electrical connector 28 remains exposed and accessible for connection to the distal connector 20D of the cable 20.

After the distal connector 20D of the cable 20 is connected to the electrical connector 28, the sleeve body 40B may be unfurled from its attached distal end 40D and extended from the cavity 30, with the proximal end 40P being drawn along the cable body 20B toward the proximal connector 20P, into the deployed configuration of **FIG. 1** and **FIG. 2****.** After completion of the procedure on the patient, the distal connector 20D of the cable 20 may be disconnected from electrical connector 28 of the control handle 16, and the cable 20 may be pulled out by its proximal end 20P from the proximal end 40P of the sleeve 40 whereupon the catheter 10 along with the attached sleeve 40 can be disposed of as a single unit.

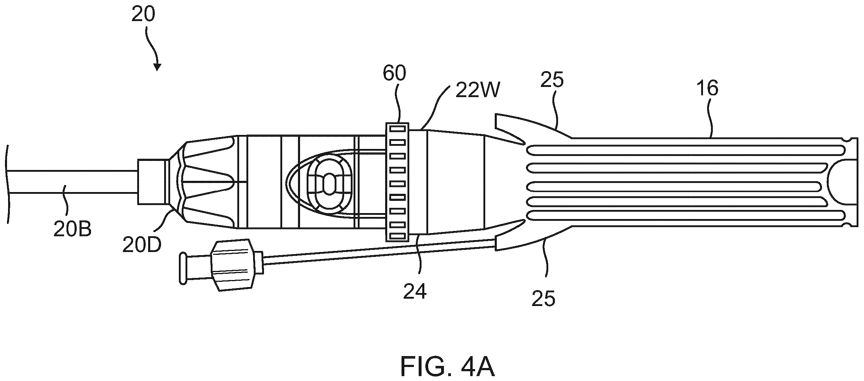

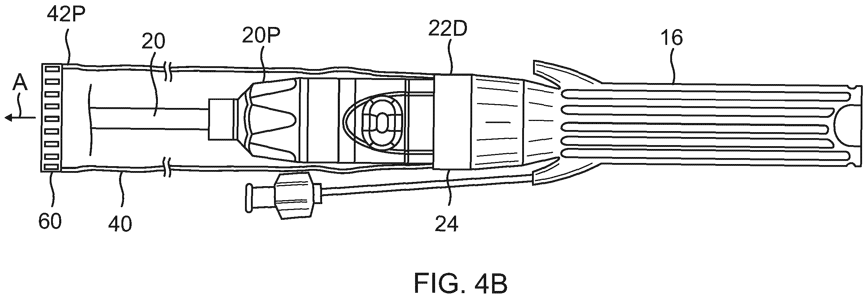

In some embodiments, the sleeve 40 includes a proximal ring member 60, as shown in **FIG. 4A** and **FIG. 4B****.** The ring member 60 is attached to and extends circumferentially around the proximal opening of the sleeve 40 and is configured to fit circumferentially on the outer surface 24 of the distal wall 22W, with the tubular body 40 tucked and stored in a toroidal or "donut" shape inside the cavity 30 of the control handle 16. After the distal connector 20D of the cable 20 is connected to the electrical connector 28 of the control handle 16 through the open center of the ring member 60 and of the through-hole of the sleeve 40 stored in a toroidal shape in the cavity 30, the tubular body 40B may be readily unfurled into the deployed configuration by the user sliding the ring member 60 proximally over the cable 20, as shown by arrow A in **FIG. 4B****.**

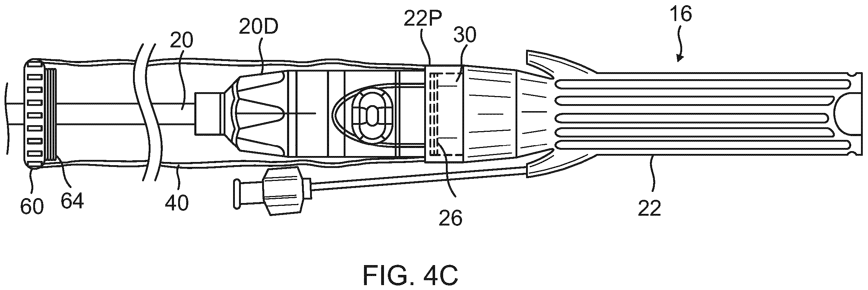

In some embodiments, as shown in **FIG. 4C****,** the proximal ring member 60 has a cylindrical threaded distal portion 64 adapted for coupling with a threaded inner surface 26 (shown in broken lines) at the proximal end 22P of the control handle 16. Prior to use, the threaded distal portion 64 is threaded into the proximal end 22P with the protective sleeve 40 tucked and stored inside the cavity 30. To deploy the protective sleeve 40, the user turns the ring member 60 relative to the control handle 16 to release the ring member 60 from the proximal end 22P, whereupon the user can slide the ring member 60 proximally over the cable 20 to unfurl the protective sleeve over the cable 20.

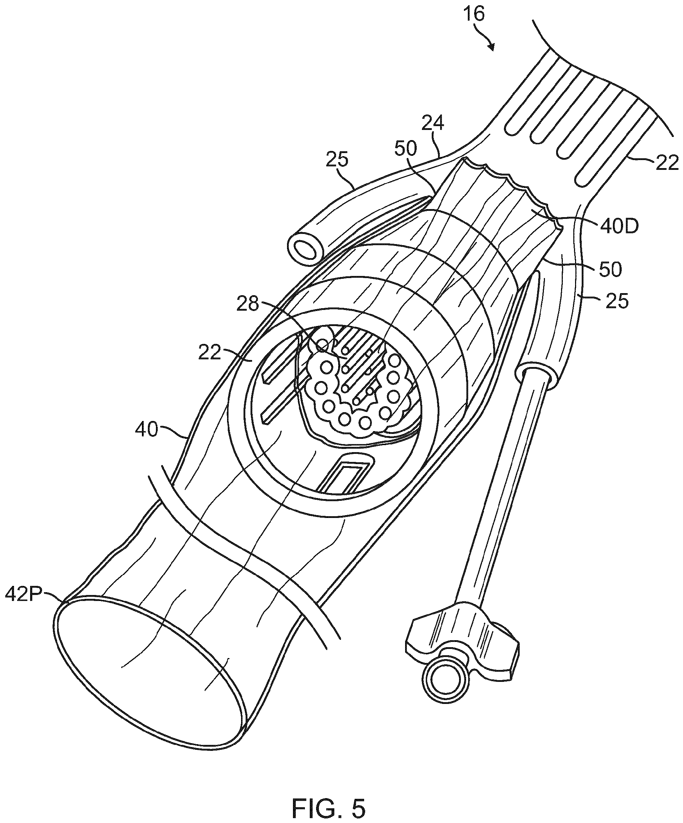

In the foregoing embodiments, the distal end of the sleeve 40 is attached to the control handle at or near the inside of the cavity 30 at the proximal end 22P. In other embodiments to be described below, the sleeve 40 is attached at its distal end 40D to the control handle 16 outside of the cavity 30. As shown in **FIG. 5****,** the distal end 40D of the sleeve 40 is mounted over the outer surface 24 of the control handle housing 22 which has slits 50 that may be formed, for example, under the arms 25. The user may first connect the cable 20 to the electrical connector 28 of the control handle 16 and then slip the distal opening of the sleeve 40 over the proximal connector 20P of the cable 20, the cable body 20B, and the proximal end 22P of the control handle 16. The user may then pull the distal end 40D toward the slits 50 and wedge the distal end 40D into the slits and anchor the distal end 40D to the control handle housing 22. It is understood that the sleeve 40 may, alternatively, be affixed to the housing 22 first, followed by the distal connector 20D of the cable 20 being fed through the sleeve 40 and connected to the electrical connector 28 of the control handle 16. After the procedure, the distal connector 20D of the cable 20 may be disconnected from the control handle 16 and the sleeve 40 may remain attached to the catheter 10 for disposal as a single unit. Alternatively, the distal end 40D of the sleeve may be pulled and released from the slits 50 to separate from the control handle 16, whereupon the sleeve 40 and the catheter 10 may be disposed of separately from each other.

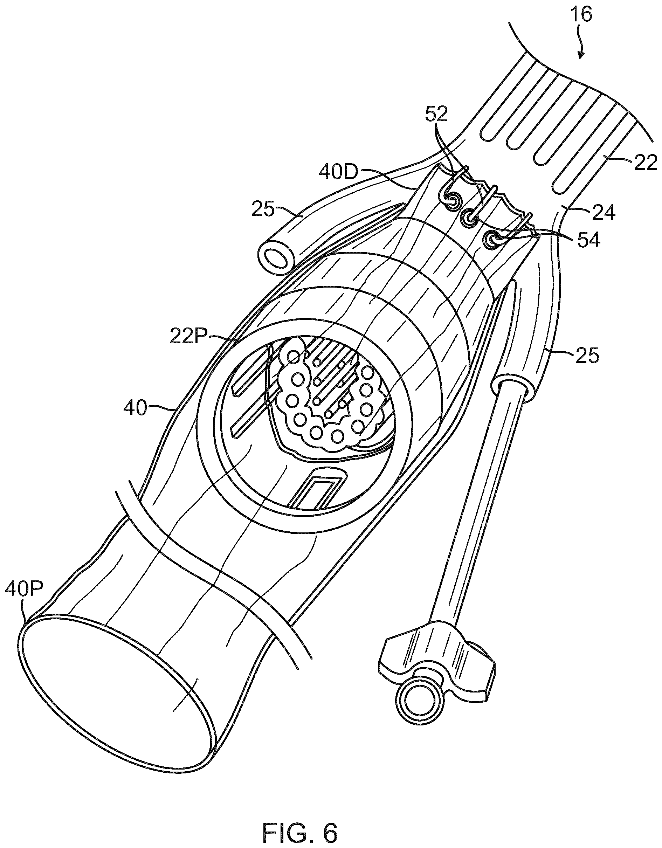

As shown in **FIG. 6****,** in some embodiments, the outer surface 24 of the housing 22 of the control handle 16 is configured with hooks 52 to which the distal end 40D of the sleeve 40 can be fastened. The distal end 40D of the sleeve 40 can be configured with preformed apertures 54, or the sleeve 40 can be pressed and punctured onto the hooks 52 such that the sleeve 40 is fastened to the hooks. After the procedure and the cable 20 is disconnected from the control handle 16, the sleeve 40 may remain attached to the catheter 10 for disposal as a single unit. Alternatively, the distal end 40D may be unhooked from the hooks 52 to separate from the control handle 16 for separate disposal.

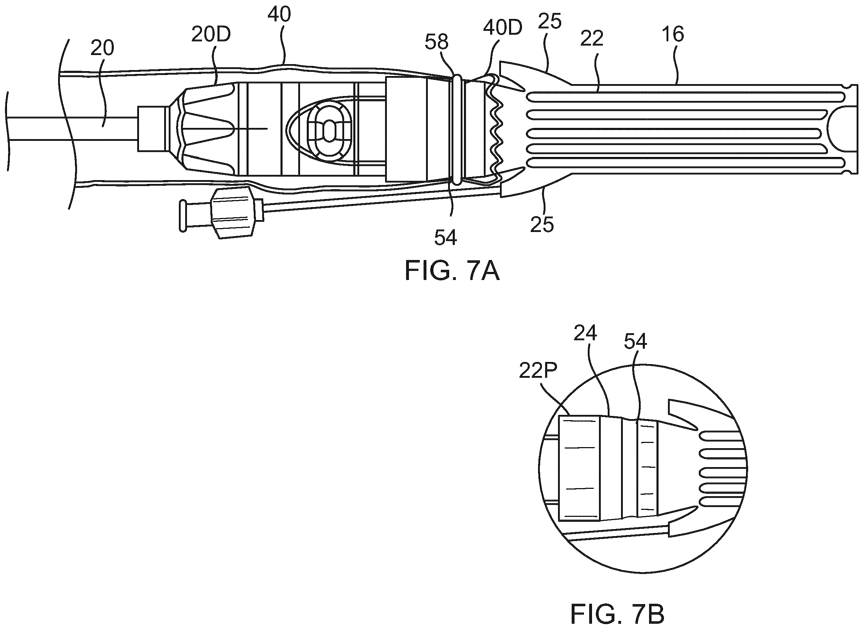

As shown in **FIG. 7A** and **FIG. 7B****,** in some embodiments, the outer surface 24 of the housing 22 is configured with a circumferential groove 54 that is covered by the distal end 40D of the sleeve 40. An elastic band 58 configured to fit snugly in the groove 54 can be positioned initially (or stored) on the housing 22 between the groove 54 and the arms 25 and then rolled over or otherwise repositioned over the distal end 40D to sit in the groove in fastening the distal end 40D of the sleeve 40 to the housing 22. After the procedure and the cable 20 is disconnected from the control handle 16, the sleeve 40 may remain attached to the catheter 10 by the elastic band 56 for disposal as a single unit. Alternatively, the elastic band 56 may be removed from the groove 54 to release the distal end 40D from the control handle 16 for separate disposal of the sleeve 40 and the control handle 16.

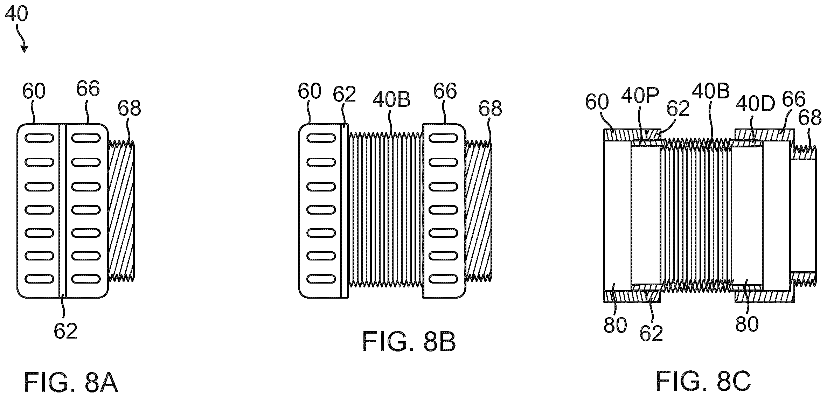

In some embodiments, a sleeve 40 assumes a stored configuration standing alone and apart from the catheter 10 and a deployed configuration after it is mounted onto the catheter 10. As shown in **FIG. 8A, FIG. 8B** and **FIG. 8C****,** the sleeve 40 includes the proximal ring member 60, the tubular body 40B, and a distal ring member 66 with a cylindrical threaded distal portion 68 where proximal end of the tubular body 40 is affixed to the proximal ring member 60 (its inner or outer surface) and the distal end of the tubular body 40 is affixed to the distal ring member 66 (its inner or outer surface). A distal surface 60D of the proximal ring member 60 includes a releasable adhesive 62 for releasable attachment to the distal ring member 66, with the tubular body 40 collapsed and packed within interiors 80 of each proximal and distal ring members 60, 66.

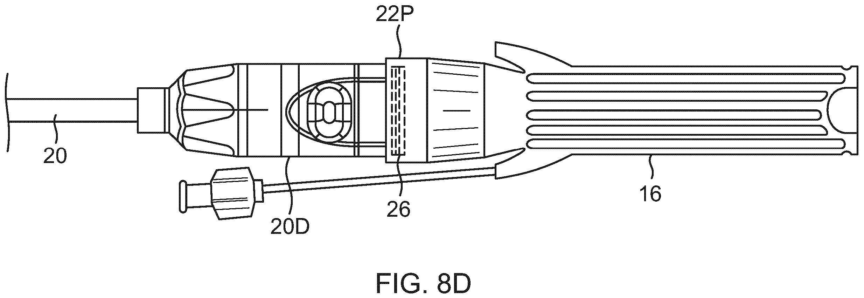

As shown in FIG. 8D, to mount the sleeve 40 onto the control handle 16 of the catheter 10, the user may thread the distal threaded insertion portion 68 to the threaded inner surface 26 of the proximal end 22P of the control handle 16. The user may then connect the distal end 20D of the cable 20 to the electrical connector 28 by feeding the distal end 20D through the proximal ring member 60, the tubular body 40 and the distal ring member 66. The user may then detach the proximal ring member 60 from the distal ring member 66 by breaking the bond of the adhesive 62 and slide the proximal ring member 62 proximally along the cable 20 toward the proximal connector 20P. At the time of disposal, the user may disconnect the distal connector 20D of the cable 20 from the electrical connector 28 of the control handle 16, slide the cable proximally out from the tubular body 40B through the proximal ring member 60 and dispose of the catheter 10 and sleeve 40 together as joined.

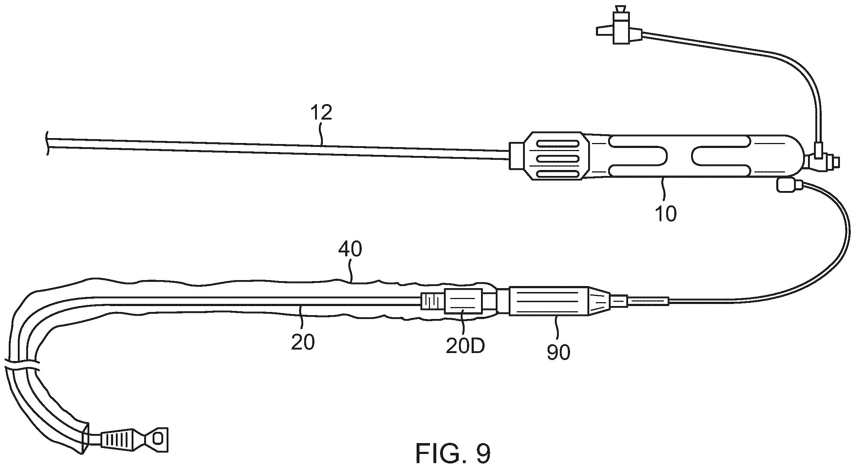

It is understood that the sleeve 40 can also be used to protect a cable 20 that connects to an electrical connector cable 90 that is connected to a catheter 10, as shown in **FIG. 9****,** with any or all of the structural elements and embodiments described above.

The sleeve 40 may be constructed of any suitable material that is fluidproof, for example, flexible thin plastic film, such as polyethylene.

The preceding description has been presented with reference to presently disclosed embodiments of the invention. Workers skilled in the art and technology to which this invention pertains will appreciate that alterations and changes in the described structure may be practiced without meaningfully departing from the principal, spirit and scope of this invention. Any feature or structure disclosed in one embodiment may be incorporated in lieu of or in addition to other features of any other embodiments, as needed or appropriate. As understood by one of ordinary skill in the art, the drawings are not necessarily to scale. Accordingly, the foregoing description should not be read as pertaining only to the precise structures described and illustrated in the accompanying drawings, but rather should be read consistent with and as support to the following claims which are to have their fullest and fair scope.

## Claims

1. A sleeve, comprising:
a distal member with a first through-hole;
a proximal member with a second through-hole; and
an elongated flexible tubular member including a distal end affixed to the distal member and a proximal end affixed to the proximal member, the tubular member including a channel therethrough, the channel in communication with the first through-hole at the distal end and in communication with the second through-hole at the proximal end.

2. The sleeve of claim 1, wherein the distal member is configured for attachment to a catheter control handle.

3. The sleeve of claim 1, wherein the distal member and the proximal member are releasably coupled to each other.

4. The sleeve of claim 1, further including a releasable adhesive between the distal member and the proximal member, or wherein the distal member includes a threaded distal portion.

5. The sleeve of claim 1, wherein i) the tubular member is constructed of a flexible thin plastic film, or ii) the tubular member is fluidproof.

6. The sleeve of claim 1, wherein each of the distal member and the proximal member has a generally circular cross-section configuration.

7. A protective sleeve configured for use with a catheter control handle, comprising:
an elongated tubular member including a distal end, a proximal end and a body portion between the distal end and the proximal end, the distal end configured for attachment to the control handle, the proximal end and the body portion configured to extend proximally from the control handle.

8. The sleeve of claim 7, further comprising an elastic loop member configured to extend circumferentially around the distal end of the sleeve and the control handle.

9. The sleeve of claim 7, wherein i) the distal end of the tubular member includes a portion configured to wedge into a slit provided in the control handle, or ii) the tubular member is constructed of a flexible thin plastic film.

10. A catheter control handle for use with an electrical cable, comprising:
a distal end electrical connector;
a housing with a longitudinal axis and proximal end that extends circumferentially around the electrical connector relative to the longitudinal axis;
an elongated sleeve including a distal end, a proximal end, and a tubular portion therebetween, the distal end affixed to the proximal end of the housing, the sleeve configured to assume a deployed configuration when the electrical cable is attached to the catheter control handle and a stored configuration when the electrical cable is detached from the catheter control handle.

11. The control handle of claim 10, wherein i) the stored configuration includes the tubular portion of the sleeve being collapsed within the proximal end of the housing, or ii) the deployed configuration includes the proximal end and the tubular portion extending proximally past the control handle.

12. The control handle of claim 10, wherein the electrical connector is configured for connection to a cable, and the sleeve in the deployed configuration includes the tubular portion and the proximal end extending proximally over at least a distal portion of the cable.

13. The control handle of claim 10, wherein the sleeve includes a construction material of a flexible thin plastic film.

14. The control handle of claim 10, wherein the proximal end includes an inner surface to which the distal end of the elongated sleeve is attached.

15. A sleeve for use with an electrical cable, the electrical cable configured for attachment to a connector cable extending from a catheter, comprising:
an elongated tubular member including a distal end, a proximal end and a middle portion extending therebetween, the distal end configured for attachment to a proximal end of the connector cable that is attached at its distal end to a control handle of the catheter, the proximal end and the middle portion of the elongated tubular member extends proximally over the electrical cable when the distal end of the electrical cable is connected to a proximal end of the connector cable, optionally wherein the sleeve includes a construction material of a flexible thin plastic film.
